# FASCICULE DE BREVET EUROPEEN

(11) **EP 2 764 344 B1**
(45) Date de publication et mention de la délivrance du brevet: **18.11.2015**
(21) Numéro de dépôt: 11770079.9
(22) Date de dépôt: 04.10.2011
(51) Int. Cl.: G01N 1/22, G01N 1/24, G01N 1/02, G01N 33/22

(54) **DISPOSITIF DE PRELEVEMENT DE POUSSIERES OU DE PARTICULES SOLIDES NOTAMMENT EN VUE DE LA DETECTION D'EXPLOSIFS**
VORRICHTUNG ZUR ENTNAHME VON STAUB- ODER FESTPARTIKELN, INSBESONDERE FÜR DEN NACHWEIS VON SPRENGSTOFFEN
DEVICE FOR SAMPLING DUST OR SOLID PARTICLES IN PARTICULAR FOR THE DETECTION OF EXPLOSIVES

(43) Date de publication de la demande: 13.08.2014
(73) Titulaire: Commissariat à l'Énergie Atomique et aux Énergies Alternatives, 75015 Paris (FR)
(72) Inventeur: BRY, Alain, 37190 VALLERES (FR); FORZY, Alexandre, F-37100 Tours (FR); HAIRAULT, Lionel, F-37150 Blere (FR)
(74) Mandataire: Ilgart, Jean-Christophe
(86) Numéro de dépôt international: PCT/EP2011/067330
(87) Numéro de publication internationale: WO 2013/050066

(56) Documents cités:
- EP-A2- 0 896 213
- WO-A1-00/16064
- DE-A1-102004 002 842
- FR-A1- 2 965 054
- US-A- 3 843 198

## Description

Le sujet de l'invention est un dispositif de prélèvement de poussières ou de particules solides, qui a été conçu en vue de la détection d'explosifs, sans que d'autres applications soient exclues, les particules prélevées pouvant être de n'importe quelle nature compatible avec les matériaux constitutifs de l'appareil.

Un procédé classique de détection de la présence de certaines substances, dont les explosifs, consiste à recourir à des chiens spécialement dressés. Mais malgré la finesse remarquable de leur odorat, l'emploi d'animaux présente les inconvénients d'une capacité de travail faible et d'une durée de dressage longue et coûteuse. C'est pourquoi d'autres procédés de détection ont été développés. Certains s'appuient sur des prélèvements d'échantillons de l'environnement qu'on analyse ensuite. La détection porte souvent sur la phase de vapeur du produit qu'on cherche à reconnaître, le prélèvement étant accompli par des dispositifs d'aspiration ; comme l'aspiration est en général à faible débit, ces dispositifs peuvent plus difficilement détecter des particules solides quand leur teneur dans l'atmosphère est faible, d'autant plus que la plupart d'entre elles peuvent adhérer à des surfaces environnantes au lieu d'être en suspension. Mais la détection de vapeurs n'est pas toujours possible : certains corps, dont de nombreux explosifs ont une pression de vapeur saturante très faible, et demeurent donc presque exclusivement à l'état solide.

C'est pourquoi il reste avantageux de prélever, en vue de leur détection, des échantillons de matières explosives sous forme solide, en poussières ou en particules. Des procédés courants sans recours à des aspirations consistent à faire un frottis de la surface à analyser pour en arracher des particules et les emporter ; mais leur emploi est interdit dans les situations fréquentes où on se refuse à toucher à des objets à analyser afin de les préserver. D'autres procédés encore font appel à des rayonnements divers pour collecter des informations visuelles, spectroscopiques ou analytiques d'explosifs, mais ceux-ci doivent être en quantité importante et sous forme massive.

Le dispositif de l'invention repose sur l'emploi d'une aspiration pour prélever des particules solides. Il se distingue de dispositifs accomplissant la même fonction principalement sous deux aspects : il ne comporte aucun actionnement électrique afin de permettre de travailler même dans des atmosphères dangereuses ou dans des températures extrêmes (par exemple -40° à +60°C), et il est conçu de manière à aspirer un gros débit même en l'absence de pompe électrique, de manière à permettre le prélèvement de particules dans un volume environnant important, ce qui permet d'en détecter même de très faibles teneurs, tout en créant une force d'aspiration suffisante pour aspirer des particules qui seraient légèrement collées sur des surfaces devant le dispositif.

Sous une forme générale, le dispositif de prélèvement conforme à l'invention comprend une poignée (1) de préhension établie sur un cyclone (2) de retenue des particules prélevées, un conduit d'air comprimé (7) responsable de l'aspiration, et une vanne mécanique (5) de fermeture du conduit d'air comprimé (7), commandée par un actionneur (6), caractérisé en ce qu'il comprend un amplificateur d'air (18), relié au cyclone (2), par un passage (3), le conduit d'air comprimé (7) débouche dans l'amplificateur d'air (8) en étant dirigé vers le passage (3), et l'amplificateur d'air (8) comprend une fente circulaire (10) dans laquelle le conduit (7) d'air comprimé débouche, et une gorge conique (12) à sommet dirigé vers le passage (3), prolongeant la fente circulaire (10) et débouchant dans un perçage (13) de l'amplificateur (8), en créant ainsi l'amplification de l'aspiration, et l'amplificateur est situé entre le cyclone et une buse (4) par laquelle les particules entrent dans le dispositif. L'actionneur peut être un bouton, une gâchette ou un robinet par exemple. La vanne étant mécanique, elle est entièrement manuelle. L'air comprimé se détendant dans la buse produit un entraînement important d'un débit de l'air extérieur qui peut être de 100% à 1000% en volumes comparables de l'air comprimé dans certaines circonstances. C'est ce qui est appelé ici l'amplification de l'aspiration et distingue nettement l'invention d'autres dispositifs où une aspiration plus classique mais moins efficace est faite. Par comparaison, US-A-3843198 décrit un dispositif où un écoulement d'air comprimé contribue à prélever un échantillon d'atmosphère, pouvant contenir des poussières ou des particules, devant un outil de coupe, par une aspiration consécutive à une dépression de l'écoulement quand il traverse un resserrement : un conduit d'aspiration s'étend du lieu du prélèvement au resserrement en passant par un cyclone où les poussières ou particules se déposent. Cette aspiration par simple dépression n'est toutefois pas apte à produire une amplification de l'aspiration, c'est-à-dire une multiplication du volume prélevé par rapport au volume d'air comprimé délivré, et d'autant moins que les pertes de charge à travers le conduit d'aspiration, assez long et occupé par le cyclone, sont importantes.

WO-A-00/16064 décrit un dispositif où l'aspiration est consécutive à un soufflage sur un cercle extérieur, ce qui permet de détacher les particules collées sur la surface de prélèvement grâce à la pression de l'air comprimé, mais aucune amplification du débit d'air comprimé n'est faite pour aspirer l'échantillon puisque c'est l'air comprimé même qui est aspiré dans le dispositif.

US-A-4 909090 et WO-A- 2009/139744 décrivent des dispositifs où l'aspiration est faite par un ventilateur, c'est-à-dire un appareil électrique, contrairement à ce qu'on souhaite ici.

Dans l'invention, le passage peut notamment contenir un tamis filtrant à grosses mailles pour arrêter des gros morceaux aspirés. Les particules prélevées et récupérées dans le cyclone sont ensuite envoyées à l'analyse.

La vanne et son actionneur (gâchette, etc.) sont installés à proximité de la poignée, et le conduit d'air comprimé est orientable. Cette disposition permet de déplacer commodément la buse devant des endroits où on souhaite procéder au prélèvement sans déplacer la source d'air, comprimé, qui est généralement une bouteille laissée à quelque distance ou portée par l'opérateur dans un sac à dos ou installée à l'intérieur de la poignée creuse pour de petits réservoirs si des faibles autonomies sont suffisantes.

L'invention sera maintenant décrite plus en détail en liaison aux figures suivantes :
- la figure 1 illustre l'invention dans son ensemble ;
- la figure 2 illustre la buse d'aspiration et l'amplificateur ;
- et la figure 3 illustre une réalisation de l'invention.

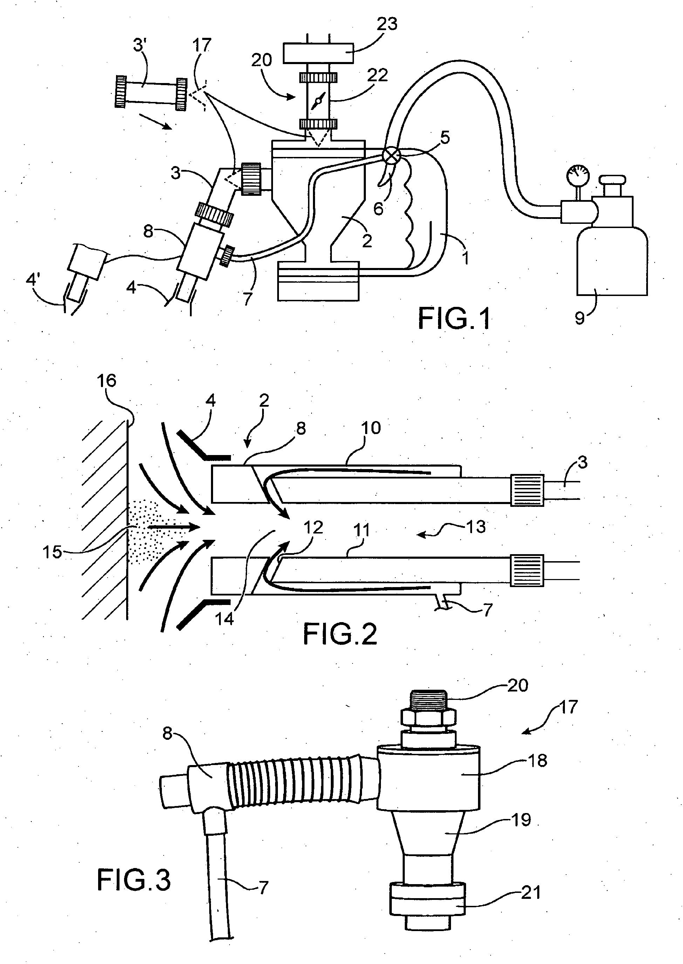

La réalisation décrite à la figure 1 comprend une poignée (1) saisie par l'opérateur. La poignée (1) est fixée sur un cyclone (2) lequel se prolonge par un passage (3) du cyclone (2) et d'un amplificateur d'air (8), auxquels le passage (3) est relié par des extrémités opposées ; le passage (3) peut aussi être orienté. Le tuyau formant le passage (3) peut être démonté de l'amplificateur d'air (8) et du cyclone (2). Les liaisons se font par des colliers de serrage, des filetages, des ajustements serrés ou d'autres moyens. La poignée (1) contient encore une vanne manuelle (5) actionnée par un bouton ou une gâchette (6) ; un tuyau souple (7) se joint à la vanne (5) positionnée à hauteur de la poignée (1) et se prolonge jusqu'à l'intérieur de l'amplificateur (8), de la façon qu'on décrira plus loin; son autre extrémité, après la vanne manuelle (5), est raccordée à une bouteille d'air comprimé (9) qui s'étend à quelque distance et peut être posée près du lieu de prélèvement, portée par un véhicule, ou par l'opérateur lui-même dans un sac à dos. Pour des petites autonomies, le gaz est contenu dans une bouteille (9) jetable ou réutilisable glissée dans l'épaisseur de la poignée (1). La vanne manuelle (5) maintient le câble souple (7) fermé au repos, mais quand la gâchette (6) est pressée, son clapet est repoussé et permet l'éjection d'air comprimé dans la buse (2). L'extrémité de l'amplificateur d'air (8) opposée au passage (3) porte une buse (4). Une sortie d'air (20) en partie supérieure du cyclone (2) est surmontée d'une vanne (22) permettant de fermer la sortie d'air (20) lorsque la gâchette (6) est pressée. Cette action force l'air à être soufflé par la buse (4) en permettant de mieux décrocher les particules avant de les aspirer. Au-dessus de la vanne (22) est positionné un réducteur de bruit (23). Des tamis filtrants (17) ou filtres sont établis aux orifices du cyclone (2), communiquant au passage (3) et à la vanne (22) menant à la sortie d'air (23). Ils sont amovibles et à mailles larges. Ils arrêtent les particules volumineuses et sont installés quand l'entrée d'impuretés, qui correspondent à ces particules, doit être empêchée ; ils peuvent aussi être retirés. Le passage (3), ici coudé pour diriger l'écoulement de l'air chargé en particules vers la paroi latérale circulaire du cyclone (2) à partir d'une zone située au pied du cyclone (2), pourrait être remplacé par un passage (3') différent, notamment droit, pour réaliser des écoulements d'air à partir d'une zone située à l'altitude du cyclone (2). De même, la buse (4), ici évasée vers cette zone extérieure d'où l'air prélevé est originaire, afin de favoriser l'aspiration, pourrait être démontée et remplacée par une autre buse (4'), notamment rétrécie vers ladite zone, afin de favoriser un soufflage par le dispositif. Les passages (3, 3', etc.) et buses (4, 4', etc.) peuvent appartenir à des jeux qui accompagnent le dispositif et sont à portée de l'opérateur. Les particules fines arrêtées par le cyclone (2) tombent dans un réservoir inférieur (21). Le réservoir inférieur (21) se démonte pour être changé facilement ou récupérer les particules qui s'y sont déposées.

La plupart des pièces du dispositif peuvent être en métal et se prêter ainsi à des nettoyages à chaud entre deux prélèvements ; mais certaines des pièces peuvent aussi être construites en matière synthétique (plastique) si on accepte de les jeter et de les remplacer après un prélèvement.

On passe au commentaire de la figure 2. L'amplificateur d'air (8) est creusé d'une fente circulaire (10) dans laquelle débouche le câble souple (7). La fente circulaire (10) s'ouvre sur la face interne (11) de la buse (2) par une gorge (12), conique avec un sommet dirigé vers le passage (3) et le cyclone (2). Quand l'air comprimé est donné, il sort de la fente circulaire (10) et de la gorge (11) en se dirigeant obliquement à travers le perçage (13) de l'amplificateur (8) puis en se redressant pour s'écouler tangentiellement à la face interne (11) par un effet Coanda. Cet écoulement crée une dépression au centre du perçage (13) à l'endroit (14), et la buse (4) est donc efficace pour aspirer l'air d'un gros volume environnant, avec une force suffisante pour décoller des particules (15) qui seraient maintenues sur une paroi (16) avoisinante, c'est-à-dire une amplification de l'aspiration. L'air et les particules entraînées passent ensuite dans le passage (3) et traversent le cyclone (2), où les particules sont retenues. Comme le cyclone (2) est en aval de l'amplificateur d'air (8) sur l'écoulement, il ne gêne pas l'amplification.

Une réalisation de l'invention est décrite à l'aide de la figure 3. Elle comprend un cyclone (2'), avec un corps périphérique (18) d'introduction du gaz, un corps cylindro-conique (19) au fond duquel les particules s'écoulent peu à peu alors que le gaz sort par une ouverture supérieure correspondant à la sortie d'air (20), et le réservoir inférieur (21), pour contenir les particules. Cette construction permet des prélèvements de particules plus fines, jusqu'à une dimension d'un micron environ. Le réservoir inférieur (21) se démonte pour être changé ou récupérer les particules qui s'y sont déposées. Le haut du corps périphérique (18) se démonte pour nettoyage.

Les essais ont montré l'efficacité de l'invention sur l'extérieur des emballages contenant des explosifs (Tolite ou Hexomax). Les durées de prélèvement ont été inférieures à la minute et l'air comprimé dépensé a été de quelques centaines de litres. Les poussières prélevées ont révélé des pics attendus dans des analyses chromatographiques.

Il est à noter que le tuyau du passage (3) peut être démonté et remplacé pour changer l'orientation d'aspiration et ainsi améliorer l'accessibilité aux particules.

## Revendications

1. Dispositif de prélèvement de particules, comprenant une poignée (1) de préhension établie sur un cyclone (2) de retenue des particules prélevées, un conduit d'air comprimé (7) responsable de l'aspiration, et une vanne mécanique (5) de fermeture du conduit d'air comprimé (7), commandée par un actionneur (6), un amplificateur d'air (18), relié au cyclone (2), par un passage (3), **caractérisé en ce que** le conduit d'air comprimé (7) débouche dans l'amplificateur d'air (8) en étant dirigé vers le passage (3), et l'amplificateur d'air (8) comprend une fente circulaire (10) dans laquelle le conduit (7) d'air comprimé débouche, et une gorge conique (12) à sommet dirigé vers le passage (3), prolongeant la fente circulaire (10) et débouchant dans un perçage (13) de l'amplificateur (8), en créant ainsi l'amplification de l'aspiration, et l'amplificateur est situé entre le cyclone et une buse (4) par laquelle les particules entrent dans le dispositif.

2. Dispositif de prélèvement de particules selon la revendication 1, **caractérisé en ce que** le moyen de retenue des particules prélevées comprend au moins un cyclone (2).

3. Dispositif de prélèvement de particules selon l'une quelconque des revendications 1 à 2, **caractérisé en ce que** la vanne (5) et l'actionneur (6) sont installés sur la poignée (1), et le conduit d'air comprimé (7) est souple.

4. Dispositif de prélèvement de particules selon l'une quelconque des revendications 1 à 3, **caractérisé en ce que** le passage (3) est formé par un tuyau qui est orientable et démontable de l'amplificateur et du moyen de prélèvement de particules.

5. Dispositif de prélèvement de particules selon la revendication 4, **caractérisé en ce que** le passage (3) est équipé d'un filtre (17) amovible à un raccordement au cyclone (2).

6. Dispositif de prélèvement de particules selon l'une quelconque des revendications 1 à 5, **caractérisé en ce que** le cyclone (2) est équipé d'une sortie d'air (20) munie d'un filtre (17) amovible et d'une vanne (22) apte à ouvrir ou fermer la sortie d'air (20).

7. Dispositif de prélèvement de particules selon la revendication 6, **caractérisé en ce que** la sortie d'air (20) est équipée d'un dispositif de réduction de bruit (23).

8. Dispositif de prélèvement de particules selon l'une quelconque des revendications 1 à 7, **caractérisé en ce que** la buse (4) est amovible de l'amplificateur d'air (8) et appartient à un jeu comprenant une buse évasée et une buse rétrécie en direction des particules à prélever.

9. Dispositif de prélèvement de particules selon l'une quelconque des revendications 1 à. 8, **caractérisé en ce que** le conduit d'air comprimé (7) aboutit à une bouteille de gaz (9).

10. Dispositif de prélèvement de particules selon l'une quelconque des revendications 1 à 9, **caractérisé en ce que** le passage (3) est coudé.

11. Dispositif de prélèvement de particules selon l'une quelconque des revendications 1 à 10, **caractérisé en ce qu'**il comprend des éléments jetables en matière synthétique.

12. Dispositif de prélèvement de particules selon l'une quelconque des revendications 1 à 11, **caractérisé en ce qu'**il comprend un réservoir inférieur (21) amovible collectant les particules prélevées.

13. Application d'un dispositif conforme à l'une quelconque des revendications précédentes à des prélèvements d'explosifs.

## Patentansprüche

1. Vorrichtung zur Entnahme von Partikeln, umfassend einen Handhabungsgriff (1), der an einem Zyklon (2) zum Zurückhalten der entnommenen Partikel vorgesehen ist, eine für die Ansaugung verantwortliche Druckluftleitung (7), sowie ein mechanisches Ventil (5) zum Schließen der Druckluftleitung (7), das von einem Betätigungselement (6) gesteuert wird, einen Luftverstärker (18), der mit dem Zyklon (2) durch einen Durchgang (3) verbunden ist, **dadurch gekennzeichnet, dass** die Druckluftleitung (7) in den Luftverstärker (8) mündet, wobei sie zum Durchgang (3) hin gerichtet ist, und dass der Luftverstärker (8) einen kreisförmigen Schlitz (10) umfasst, in den die Druckluftleitung (7) mündet, sowie eine konische Auskehlung (12) mit einer Spitze, die zum Durchgang (3) hin gerichtet ist, die den kreisförmigen Schlitz (10) verlängert und in eine Bohrung (13) des Verstärkers (8) mündet, wodurch somit die Verstärkung der Ansaugung erzeugt wird, und dass der Verstärker zwischen dem Zyklon und einer Düse (4) angeordnet ist, durch die die Partikel in die Vorrichtung eintreten.

2. Vorrichtung zur Entnahme von Partikeln nach Anspruch 1, **dadurch gekennzeichnet, dass** die Einrichtung zum Zurückhalten der entnommenen Partikel wenigstens einen Zyklon (2) umfasst.

3. Vorrichtung zur Entnahme von Partikeln nach einem der Ansprüche 1 bis 2, **dadurch gekennzeichnet, dass** das Ventil (5) und das Betätigungselement (6) an dem Griff (1) installiert sind, und dass die Druckluftleitung (7) flexibel ist.

4. Vorrichtung zur Entnahme von Partikeln nach einem der Ansprüche 1 bis 3, **dadurch gekennzeichnet, dass** der Durchgang (3) durch einen Schlauch gebildet ist, der orientierbar und vom Verstärker sowie von der Einrichtung zur Entnahme von Partikeln demontierbar ist.

5. Vorrichtung zur Entnahme von Partikeln nach Anspruch 4, **dadurch gekennzeichnet, dass** der Durchgang (3) mit einem Filter (17) ausgestattet ist, der lösbar an einem Anschluss am Zyklon (2) anbringbar ist.

6. Vorrichtung zur Entnahme von Partikeln nach einem der Ansprüche 1 bis 5, **dadurch gekennzeichnet, dass** der Zyklon (2) mit einem Luftauslass (20) ausgestattet ist, der mit einem lösbaren Filter (17) versehen ist, sowie mit einem Ventil (22), das dazu ausgelegt ist, den Luftauslass (20) zu öffnen oder zu schließen.

7. Vorrichtung zur Entnahme von Partikeln nach Anspruch 6, **dadurch gekennzeichnet, dass** der Luftauslass (20) mit einer Vorrichtung zur Lärmreduktion (23) ausgestattet ist.

8. Vorrichtung zur Entnahme von Partikeln nach einem der Ansprüche 1 bis 7, **dadurch gekennzeichnet, dass** die Düse (4) vom Luftverstärker (8) lösbar ist und zu einer Gruppe gehört, die eine aufgeweitete Düse und eine verengte Düse in Richtung der zu entnehmenden Partikel umfasst.

9. Vorrichtung zur Entnahme von Partikeln nach einem der Ansprüche 1 bis 8, **dadurch gekennzeichnet, dass** die Druckluftleitung (7) an einer Gasflasche (9) endet.

10. Vorrichtung zur Entnahme von Partikeln nach einem der Ansprüche 1 bis 9, **dadurch gekennzeichnet, dass** der Durchgang (3) gebogen ist.

11. Vorrichtung zur Entnahme von Partikeln nach einem der Ansprüche 1 bis 10, **dadurch gekennzeichnet, dass** sie wegwerfbare Elemente aus Kunststoffmaterial umfasst.

12. Vorrichtung zur Entnahme von Partikeln nach einem der Ansprüche 1 bis 11, **dadurch gekennzeichnet, dass** sie ein lösbares unteres Reservoir (21) umfasst, welches die entnommenen Partikel aufsammelt.

13. Verwendung einer Vorrichtung gemäß einem der vorhergehenden Ansprüche bei der Entnahme von Sprengstoffen.

## Claims

1. Device for sampling particles, comprising a handle (1) for grasping established on a cyclone (2) for retaining sampled particles, a compressed air duct (7) responsible for the suction, and a mechanical valve (5) for closing the compressed air duct (7), controlled by an actuator (6), comprising an air amplifier (18), connected to the cyclone (2), via a passage (3), **characterised in that** the compressed air duct (7) opens into the air amplifier (8) by being directed towards the passage (3), and the air amplifier (8) comprises a circular slot (10) wherein the compressed air duct (7) opens, and a tapered groove (12) with a top directed towards the passage (3), extending the circular slot (10) and opening into a bore (13) of the amplifier (8), as such creating the amplification of the suction, and the amplifier is located between the cyclone and a nozzle (4) through which the particles enter into the device.

2. Device for sampling particles according to claim 1, **characterised in that** the means for retaining sampled particles comprises at least one cyclone (2).

3. Device for sampling particles according to any of claims 1 to 2, **characterised in that** the valve (5) and the actuator (6) are installed on the handle (1), and the compressed air duct (7) is flexible.

4. Device for sampling particles according to any of claims 1 to 3, **characterised in that** the passage (3) is formed by a hose which can be oriented and which can be dismounted from the amplifier and from the means for sampling particles.

5. Device for sampling particles according to claim 4, **characterised in that** the passage (3) is provided with a movable filter (17) with a connection to the cyclone (2).

6. Device for sampling particles according to any of claims 1 to 5, **characterised in that** the cyclone (2) is provided with an air outlet (20) provided with a movable filter (17) and with a valve (22) capable of opening or closing the air outlet (20).

7. Device for sampling particles according to claim 6, **characterised in that** the air outlet (20) is provided with a device for reducing noise (23).

8. Device for sampling particles according to any of claims 1 to 7, **characterised in that** the nozzle (4) is movable from the air amplifier (8) and belongs to a set comprising a flared nozzle and a tapered nozzle in the direction of the particles to be sampled.

9. Device for sampling particles according to any of claims 1 to 8, **characterised in that** the compressed air duct (7) exits into a bottle of gas (9).

10. Device for sampling particles according to any of claims 1 to 9, **characterised in that** the passage (3) is bent.

11. Device for sampling particles according to any of claims 1 to 10, **characterised in that** it comprises elements that can be discarded made from synthetic material.

12. Device for sampling particles according to any of claims 1 to 11, **characterised in that** it comprises a lower movable reservoir (21) that collects the sampled particles.

13. Application of a device according to any of the previous claims to samplings of explosives.
